# EUROPEAN PATENT APPLICATION

(11) **EP 4 803 014 A1**
(43) Date of publication of application: **09.09.2026**
(21) Application number: 25162078.7
(22) Date of filing: 06.03.2025
(51) Int. Cl.: A61B 8/08, A61B 8/00

(54) **STANDARD IMAGING PLANE DETECTION IN ULTRASOUND IMAGING WITH BLIND SWEEP PROTOCOL**

(71) Applicant: Koninklijke Philips N.V., 5656 AE Eindhoven (NL)
(72) Inventor: PATEL, Priyam, Eindhoven (NL)
(74) Representative: Philips Intellectual Property & Standards

(57) **Abstract**

An apparatus includes a processor configured for communication with an ultrasound probe. The processor is configured to control the ultrasound probe to obtain a first group of ultrasound image frames during one or more sweeps of a blind sweep protocol on a patient with a pregnancy, and determine, based on the first group of ultrasound image frames, a location of the patient to perform a follow-up scan with the ultrasound probe, where the location is associated with a first fetal anatomy used to determine the gestational age of a fetus. The processor is also configured to control the ultrasound probe to obtain a second group of ultrasound image frames during the follow-up scan at the location; identify an ultrasound image frame in the second group that includes a pre-defined imaging plane of the first fetal anatomy; determine the gestational age using the ultrasound image frame; and output the gestational age.

## Description

### FIELD OF THE INVENTION

The subject matter described herein relates to devices, systems, and methods for using ultrasound data from a blind abdominal imaging sweeps to detect a standard imaging plane for fetal gestational age measurement.

### BACKGROUND OF THE INVENTION

Ultrasound imaging is often used for diagnostic purposes in an office or hospital setting, but may also be used in resource-constrained care settings (e.g., homes, accident sites, ambulances, mobile health facilities, etc.) by emergency personnel, home health nurses, midwives, etc., who may lack ultrasound expertise. Ultrasound imaging is a vital component of high-quality obstetric care. For example, estimation of the gestational age of the fetus is critical for predicting fetal development, identifying potential complications, and planning for delivery. The widely used Hadlock formula/table is used to estimate gestational age based on measurements of specific fetal anatomies, such as the head, femur, and abdomen, obtained from standard plane images. Proper measurement can allow for appropriate referral for delivery care in highly resourced centers with providers trained to handle the complications. However, in rural and under-resourced communities, the scarcity of ultrasound imaging results in a considerable gap in the healthcare of pregnant mothers.

To facilitate ultrasound image acquisition by untrained or minimally trained users, a "blind sweep" protocol is often employed, in which the user follows pre-determined probe paths (e.g., sweeping out a pattern on the patient's abdomen) to capture fetal anatomical structures. However, these blind sweeps rarely contain standard imaging planes obtained by an experienced user. The experienced user takes fetal anatomical measurements in these standard planes for gestational age estimation of a fetus. Thus, blind sweeps may not provide the necessary imaging planes required for the Hadlock formula. As a result, gestational age estimation in this scenario relies heavily on end-to-end deep learning methods (which provide little insight to clinicians and therefore may not be fully trusted by clinicians), or is often not computed at all.

The information included in this Background section of the specification, including any references cited herein and any description or discussion thereof, is included for technical reference purposes only and is not to be regarded as subject matter by which the scope of the disclosure is to be bound.

### SUMMARY OF THE INVENTION

The invention is defined by the independent claims. Advantageous embodiments are provided in the dependent claims. Disclosed is an ultrasound sweep standard plane detection system that, following a blind sweep protocol, detects anatomical features in the captured images and uses them to guide novice users to re-scan areas of the abdomen in order to detect standard measurement planes. A front-end anatomy detector identifies anatomies of interest, followed by a series of acquisitions with visual or audio prompts to help the user locate and measure standard imaging planes. A backend standard plane detector neural network then measures the planes, enabling accurate gestational age estimation. Aspects of the present disclosure advantageously identify one or multiple standard planes in the context of the blind sweep protocol (which typically does not rely on standard planes).

A system of one or more computers can be configured to perform particular operations or actions by virtue of having software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions.

One general aspect includes an apparatus with a processor circuit configured for communication with an ultrasound probe, where the processor circuit is configured to: control the ultrasound probe to obtain a first plurality of ultrasound image frames during one or more sweeps of a blind sweep protocol on a patient with a pregnancy; determine, based on the first plurality of ultrasound image frames, a location of the patient to perform a follow-up scan with the ultrasound probe, where the location is associated with a first fetal anatomy used to determine a gestational age of a fetus; control the ultrasound probe to obtain a second plurality of ultrasound image frames during the follow-up scan at the location; identify an ultrasound image frame in the second plurality may include a pre-defined imaging plane of the first fetal anatomy; determine the gestational age using the ultrasound image frame; and output, to a display in communication with the processor, the gestational age. Other embodiments of this aspect include corresponding computer systems, apparatus, and computer programs recorded on one or more computer storage devices, each configured to perform the actions of the methods. In particular, another general aspect includes a method for determining a gestational age of a fetus using ultrasound data from blind abdominal imaging sweeps, comprising: controlling an ultrasound probe to obtain a first plurality of ultrasound image frames during one or more sweeps of a blind sweep protocol on a patient with a pregnancy; determining, based on the first plurality of ultrasound image frames, a location of the patient to perform a follow-up scan with the ultrasound probe, wherein the location is associated with a first fetal anatomy used to determine a gestational age of a fetus; controlling the ultrasound probe to obtain a second plurality of ultrasound image frames during the follow-up scan at the location;
identifying an ultrasound image frame in the second plurality comprising a pre-defined imaging plane of the first fetal anatomy; determining the gestational age using the ultrasound image frame; and outputting, to a display, the gestational age. Another general aspect includes a non-transitory computer-readable medium storing instructions that, when executed by a processor, cause the processor to perform the above method.

Implementations may include one or more of the following features. In some aspects, to determine the location of the patient to perform the follow-up scan, the processor circuit is configured to: provide the plurality of ultrasound image frames as an input to a deep learning network trained to detect a plurality of fetal anatomies; and generate, as an output of the deep learning network, a plurality of detections of the plurality of fetal anatomies within the plurality of ultrasound image frames, wherein the first fetal anatomy may include one of the plurality of fetal anatomies, wherein the plurality of detections may include a detection of the first fetal anatomy, and wherein the location may include a location of the detection of the first fetal anatomy. In some aspects, the processor circuit is configured to: output, to a display in communication with the processor circuit, a user instruction to perform the follow-up scan with the ultrasound probe at the location. In some aspects, the processor circuit is configured to generate a map of the plurality of detections, wherein the user instruction to the user may include the map. In some aspects, the map may include a plurality of regions that are visually distinguished from one another and representative of different fetal anatomies. In some aspects, the processor is configured to output a user instruction to perform a plurality of movements with the ultrasound probe during the follow-up scan, wherein the plurality of movements may include at least one of rocking the ultrasound, fanning the ultrasound probe, or rotating the ultrasound probe. In some aspects, the one or more sweeps of the blind sweep protocol do not include the at least one of rocking the ultrasound probe, fanning the ultrasound probe, or rotating the ultrasound probe. In some aspects, to identify that the ultrasound image frame may include the pre-defined imaging plane, the processor circuit is configured to: provide the second plurality of ultrasound image frames as an input to a deep learning network trained to detect one or more pre-defined imaging planes; and generate, as an output of the deep learning network, a detection that the ultrasound image may include the pre-defined imaging plane. In some aspects, in response to identification of the ultrasound image frame, the processor circuit is configured to output a user instruction that may include at least one of an indication that the pre-defined imaging plane has been found or guidance to end the follow-up scan. In some aspects, the first fetal anatomy may include one of a fetal head, a fetal abdomen, or a fetal femur. In some aspects, to determine the gestational age, the processor is configured to use: a measurement of a head circumference or a measurement of a biparietal diameter in the ultrasound image frame, when the first fetal anatomy may include the fetal head; a measurement of an abdominal circumference in the ultrasound image frame, when the first fetal anatomy may include the fetal abdomen; or a measurement of a femur length in the ultrasound image frame, when the first fetal anatomy may include the fetal femur. In some aspects, the processor circuit is configured to automatically perform at least one the measurement of the head circumference, the measurement of the biparietal diameter, the measurement of the abdominal circumference, or the measurement of the femur length. In some aspects, the processor circuit is configured to: determine, based on the first plurality of ultrasound image frames, a further location of the patient to perform a further follow-up scan with the ultrasound probe, wherein the further location is associated with a different, second fetal anatomy used to determine the gestational age; control the ultrasound probe to obtain a third plurality of ultrasound image frames during the further follow-up scan at further location; identify a further ultrasound image frame in the third plurality may include a pre-defined imaging plane of the second fetal anatomy; determine the gestational age using the further ultrasound image frame. In some aspects, the first fetal anatomy and the second fetal anatomy each may include a different one of: a fetal head, a fetal abdomen, or a fetal femur. In some aspects, the apparatus may include the ultrasound probe. Implementations of the described techniques may include hardware, a method or process, or computer software on a computer-accessible medium.

One general aspect includes an apparatus. The apparatus includes a processor circuit configured for communication with an ultrasound probe, where the processor circuit is configured to: control the ultrasound probe to obtain a plurality of ultrasound image frames during one or more random sweeps on a patient with a pregnancy; identify a first ultrasound image frame in the plurality may include a pre-defined imaging plane of first fetal anatomy; determine the gestational age using a measurement of the first fetal anatomy in the first ultrasound image frame; and output, to a display in communication with the processor, the gestational age. Other embodiments of this aspect include corresponding computer systems, apparatus, and computer programs recorded on one or more computer storage devices, each configured to perform the actions of the methods.

In some aspects, to identify that the first ultrasound image frame may include the pre-defined imaging plane, the processor circuit is configured to: provide the first plurality of ultrasound image frames as an input to a deep learning network trained to detect one or more pre-defined imaging planes; and generate, as an output of the deep learning network, a detection that the ultrasound image may include the pre-defined imaging plane. In some aspects, the processor circuit is configured to: identify that a second ultrasound image frame in the plurality may include a pre-defined imaging plane of second fetal anatomy; determine the gestational age using a measurement of the second fetal anatomy in the second ultrasound image frame. In some aspects, the first fetal anatomy and the second fetal anatomy each may include a different one of: a fetal head, a fetal abdomen, or a fetal femur. In some aspects, the apparatus may include the ultrasound probe. Implementations of the described techniques may include hardware, a method or process, or computer software on a computer-accessible medium.

This Summary is provided to introduce a selection of concepts in a simplified form that are further described below in the Detailed Description. This Summary is not intended to identify key features or essential features of the claimed subject matter, nor is it intended to limit the scope of the claimed subject matter. A more extensive presentation of features, details, utilities, and advantages of the ultrasound sweep standard plane detection system, as defined in the claims, is provided in the following written description of various aspects of the disclosure and illustrated in the accompanying drawings.

### BRIEF DESCRIPTION OF THE DRAWINGS

Illustrative aspects of the present disclosure will be described with reference to the accompanying drawings, of which:
Fig. 1 is a schematic, diagrammatic representation of an ultrasound imaging system, according to aspects of the present disclosure.
Fig. 2 is a schematic diagram of a processor circuit, according to aspects of the present disclosure.
Fig. 3 is a schematic, diagrammatic representation of a patient, according to aspects of the present disclosure.
Fig. 4 is a schematic, diagrammatic representation, in hybrid flow diagram / block diagram form, of an example present-day GA measurement workflow for an experienced user, according to aspects of the present disclosure.
Fig. 5 is a schematic, diagrammatic representation, in hybrid flow diagram / block diagram form, of an example present-day GA measurement workflow for an inexperienced user, according to aspects of the present disclosure.
Fig. 6 is a schematic, diagrammatic representation of a fetal head, according to aspects of the present disclosure.
Fig. 7 is a schematic, diagrammatic representation of a fetal head, according to aspects of the present disclosure.
Fig. 8 is a schematic, diagrammatic representation, in hybrid block diagram/flow diagram form, of an example ultrasound sweep standard plane detection system, according to aspects of the present disclosure.
Fig. 9 is a schematic, diagrammatic representation, in hybrid block diagram/flow diagram form, of an example ultrasound sweep anatomy detection method to determine fetal gestational age, according to aspects of the present disclosure.
Fig. 10 is a schematic, diagrammatic representation, in flow diagram form, of an example standard plane imaging method, according to aspects of the present disclosure.
Fig. 11A is a schematic, diagrammatic overview, in block diagram form, of a training mode for an untrained neural network, according to aspects of the present disclosure.
Fig. 11B is a schematic, diagrammatic overview, in block diagram form, of an inference mode or clinical usage mode for the trained neural network, according to aspects of the present disclosure.
Fig. 12 is a schematic, diagrammatic illustration, in block diagram form, of the detection of anatomy (e.g., the fetal head, abdomen, femur, etc.), according to aspects of the present disclosure.
Fig. 13 is a schematic, diagrammatic representation of the ultrasound image frames of a horizontal blind sweep (e.g., a C1, C2, or C3 sweep), according to aspects of the present disclosure.
Fig. 14 is a screen display of an example ultrasound sweep standard plane detection system, according to aspects of the present disclosure.
Fig. 15 is a screen display of an example ultrasound sweep standard plane detection system, according to aspects of the present disclosure.
Fig. 16 is a schematic, diagrammatic representation, in flow diagram form, of an example ultrasound sweep standard plane detection method, according to aspects of the present disclosure.
Fig. 17 is a screen display of an example ultrasound sweep standard plane detection system, according to aspects of the present disclosure.
Fig. 18 is a screen display of an example ultrasound sweep standard plane detection system, according to aspects of the present disclosure.
Fig. 19 is a screen display of an example ultrasound sweep standard plane detection system, according to aspects of the present disclosure.
Fig. 20 is a schematic, diagrammatic representation of the standard measurement plane of a fetal head, according to aspects of the present disclosure.
Fig. 21 is a schematic, diagrammatic representation of a patient, according to aspects of the present disclosure.
Fig. 22 is a schematic, diagrammatic representation, in flow diagram form, of an example ultrasound sweep standard plane detection method, according to aspects of the present disclosure.

### DETAILED DESCRIPTION OF EMBODIMENTS

In accordance with at least one aspect of the present disclosure, an ultrasound sweep standard plane detection system is provided which, following a blind sweep protocol (sweeps at particular locations along body without trying to find specific anatomy), detects anatomical features in the captured images and uses them to guide novice users, such as nurses or midwives, to re-scan certain specific areas of the abdomen in order to detect standard planes for measurement in blind sweeps. This approach involves using a front-end anatomy detector to identify anatomies of interest. For example, a deep learning network, such as a convolutional neural network, is used to detect anatomy, such as the head, abdomen, femur, etc., or landmark portions thereof.

This detection is followed by a series of acquisitions with visual or audio prompts to help the user locate and measure one or more standard imaging planes. A backend standard plane detector neural network then measures the planes, enabling accurate gestational age estimation (e.g., using the Hadlock formula). This guided workflow has the potential to improve gestational age estimation accuracy and user experience, particularly in resource-constrained settings where point-of-care (POC) scanners are used.

A benefit of the ultrasound sweep standard plane detection system is to improve the quality of care by using information obtained during the blind sweep protocol to identify the standard planes used for gestational age measurement.

Problems or disadvantages overcome by the ultrasound sweep standard plane detection system include:
Difficulty in Gestational Age Estimation: Blind sweeps, which involve moving the probe in vertical and horizontal sweeps along the mother's abdomen, do not provide all the standard plane images necessary for gestational age estimation using the Hadlock formula. This presents challenges to accurately estimating gestational age.

Lack of Standard Planes: Blind sweeps may not capture all standard planes required for gestational age estimation, leading to incomplete or inaccurate measurements.

Dependence on End-to-End Deep Learning Methods: Without standard plane images, gestational age estimation relies on end-to-end deep learning methods, which can be complex, time-consuming, and may not always provide accurate results.

Ease of standard plane acquisition for novice users: Novice users are not trained to acquire standard plane images. However, with the ultrasound sweep standard plane detection system, they can acquire standard plane images to then estimate the gestational age.

The present disclosure provides devices, systems, and methods to guide a novice user like a nurse or midwife to detect standard planes for gestational age (GA) measurement. First, after acquisition of blind sweeps, the anatomy detector detects anatomies of interest. An anatomy grid is processed and shown on screen, and the user is (e.g., visually or via audio) prompted to place the probe again in the approximate location(s) shown on the screen. The user then does a series of acquisitions, such as rotating the probe, translating the probe, and/or tilting the probe around that area.

When the backend standard plane detector neural network detects standard measurement planes, the user stops acquiring, and the standard plane is then measured via a neural network or image processing algorithm.

A first aspect includes one or more follow-up scans based on an anatomy map. In this aspect, the system takes the blind sweep data and passes it through the fetal anatomy detector, which detects the location of a number of fetal anatomies (e.g., the head, abdomen, and/or femur, used in the Hadlock formula). The fetal anatomy detector could be any object detection or segmentation model. The anatomy detections are then plotted onto an anatomy map grid to show the approximate location and orientation of the fetus. The user is then prompted by A/V prompts through the user interface (UI) to perform a series of acquisitions based on the anatomy map grid. The prompts could be something like "Navigate to the top left part of the mother's abdomen and then rotate the probe 30 degrees."

The backend standard plane detector model continuously checks for standard planes while the user is acquiring the sweep. The standard plane detector could be any classification or object detection model. When the standard plane is acquired, the measurement is done on the plane by the model itself, or by manually drawing by the user.

A second aspect includes UI-based guidance elements, such as any UI that displays an anatomy map grid, any UI that guides the user via an audio-visual prompt, and/or any UI that prompts the user to stop scanning or displays a message such as "Standard plane found", or equivalents thereof.

A third aspect includes random sweeping of the abdomen until one or more standard planes are acquired (e.g., no stopping prompts and no displayed anatomy grid; instead just sweeping the abdomen randomly). The system may then prompt the user to stop sweeping once the standard plane(s) have been detected and measured.

Aspects of the present application (e.g., a deep learning model trained to estimate a clinical feature) can include or be similar to those described in U.S. Provisional Application No. 63/611,810, filed December 19, 2023, titled "Low-Lying Placenta and/or Placenta Location in Ultrasound Imaging With Blind Sweep Protocol", U.S. Provisional Application No. 63/540,755, filed September 27, 2023, titled "Ultrasound Imaging With Follow Up Sweep Guidance After Blind Sweep Protocol", U.S. Provisional Application No. 63/655,754, filed June 4, 2024, titled "Multiple Pregnancy/Gestation Detection Based On Graphing And Skeletonization Of Fetal Anatomy Detections From Ultrasound Imaging Blind Sweep Protocol", U.S. Provisional Application No. 63/620,385, filed January 12, 2024, titled "Multiple Pregnancy/Gestation Detection Using Ultrasound Imaging With Blind Sweep Protocol", and U.S. Provisional Application No. 63/540,740, filed September 27, 2023, titled "Ultrasound Imaging With Ultrasound Probe Guidance In Blind Sweep Protocol", and U.S. Provisional Application No. 63/690,889, filed September 5, 2024, titled "Ultrasound-Based Verification Of Uterine Imaging Extent Using Blind Sweep Protocol", each of which is incorporated by reference herein.

The present disclosure aids substantially in the capture of high-quality ultrasound images by minimally trained users, by detecting the standard measurement planes that are used for gestational age measurement. Implemented on a processor in communication with an ultrasound probe, the ultrasound sweep standard plane detection system disclosed herein provides practical improvements in the quality of care available to patients in underserved areas. This improved imaging methodology transforms a process that is heavily reliant on professional experience into one that is accurate and repeatable even for minimally trained personnel, without the normally routine need to train clinicians such as midwives to locate standard planes. This unconventional approach improves the functioning of the ultrasound imaging system, by providing reliable, repeatable imaging in hospital, office, vehicle, field, and home settings, as well as referral recommendations for patients suspected to have a pregnancy complication.

The ultrasound sweep standard plane detection system may be implemented as a process at least partially viewable on a display, and operated by a control process executing on a processor that accepts user inputs from a keyboard, mouse, or touchscreen interface, and that is in communication with one or more sensor probes. In that regard, the control process performs certain specific operations in response to different inputs or selections made at different times. Certain structures, functions, and operations of the processor, display, sensors, and user input systems are known in the art, while others are recited herein to enable novel features or aspects of the present disclosure with particularity.

These descriptions are provided for exemplary purposes only, and should not be considered to limit the scope of the ultrasound sweep standard plane detection system. Certain features may be added, removed, or modified without departing from the spirit of the claimed subject matter.

For the purposes of promoting an understanding of the principles of the present disclosure, reference will now be made to the aspects illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one aspect may be combined with the features, components, and/or steps described with respect to other aspects of the present disclosure. For the sake of brevity, however, the numerous iterations of these combinations will not be described separately.

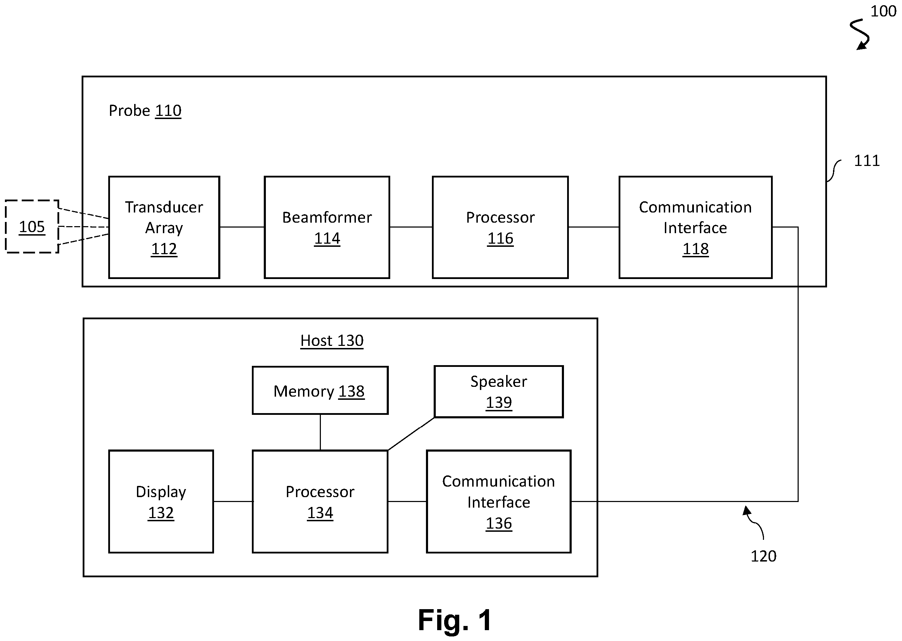

Fig. 1 is a schematic, diagrammatic representation of an ultrasound imaging system 100, according to aspects of the present disclosure. The ultrasound imaging system 100 may for example be used to acquire ultrasound video sweeps, which can then be analyzed by a human clinician or an artificial intelligence to diagnose medical conditions.

The ultrasound imaging system 100 is used for scanning an area or volume of a subject's body. A subject may include a patient of an ultrasound imaging procedure, or any other person, or any suitable living or non-living organism or structure. The ultrasound imaging system 100 includes an ultrasound imaging probe 110 in communication with a host 130 over a communication interface or link 120. The probe 110 may include a transducer array 112, a beamformer 114, a processor circuit 116, and a communication interface 118. The host 130 may include a display 132, a processor circuit 134, a communication interface 136, and a memory 138 storing subject information.

In some aspects, the probe 110 is an external ultrasound imaging device including a housing 111 configured for handheld operation by a user. The transducer array 112 can be configured to obtain ultrasound data while the user grasps the housing 111 of the probe 110 such that the transducer array 112 is positioned adjacent to or in contact with a subject's skin. The probe 110 is configured to obtain ultrasound data of anatomy within the subject's body while the probe 110 is positioned outside of the subject's body for general imaging, such as for abdomen imaging, liver imaging, etc. In some aspects, the probe 110 can be an external ultrasound probe, a transthoracic probe, and/or a curved array probe.

In other aspects, the probe 110 can be an internal ultrasound imaging device and may comprise a housing 111 configured to be positioned within a lumen of a subject's body for general imaging, such as for abdomen imaging, liver imaging, etc. In some aspects, the probe 110 may be a curved array probe. Probe 110 may be of any suitable form for any suitable ultrasound imaging application including both external and internal ultrasound imaging.

Some aspects of the present disclosure can be implemented with medical images of subjects obtained using any suitable medical imaging device and/or modality. Examples of medical images and medical imaging devices include x-ray images (angiographic images, fluoroscopic images, images with or without contrast) obtained by an x-ray imaging device, computed tomography (CT) images obtained by a CT imaging device, positron emission tomography-computed tomography (PET-CT) images obtained by a PET-CT imaging device, magnetic resonance images (MRI) obtained by an MRI device, single-photon emission computed tomography (SPECT) images obtained by a SPECT imaging device, optical coherence tomography (OCT) images obtained by an OCT imaging device, and intravascular photoacoustic (IVPA) images obtained by an IVPA imaging device. The medical imaging device can obtain the medical images while positioned outside the subject body, spaced from the subject body, adjacent to the subject body, in contact with the subject body, and/or inside the subject body.

For an ultrasound imaging device, the transducer array 112 emits ultrasound signals towards an anatomical object 105 of a subject and receives echo signals reflected from the object 105 back to the transducer array 112. The ultrasound transducer array 112 can include any suitable number of acoustic elements, including one or more acoustic elements and/or a plurality of acoustic elements. In some instances, the transducer array 112 includes a single acoustic element. In some instances, the transducer array 112 may include an array of acoustic elements with any number of acoustic elements in any suitable configuration. For example, the transducer array 112 can include between 1 acoustic element and 10000 acoustic elements, including values such as 2 acoustic elements, 4 acoustic elements, 36 acoustic elements, 64 acoustic elements, 128 acoustic elements, 500 acoustic elements, 812 acoustic elements, 1000 acoustic elements, 3000 acoustic elements, 8000 acoustic elements, and/or other values both larger and smaller. In some instances, the transducer array 112 may include an array of acoustic elements with any number of acoustic elements in any suitable configuration, such as a linear array, a planar array, a curved array, a curvilinear array, a circumferential array, an annular array, a phased array, a matrix array, a one-dimensional (1D) array, a 1.x dimensional array (e.g., a 1.5D array), or a two-dimensional (2D) array. The array of acoustic elements (e.g., one or more rows, one or more columns, and/or one or more orientations) can be uniformly or independently controlled and activated. The transducer array 112 can be configured to obtain one-dimensional, two-dimensional, and/or three-dimensional images of a subject's anatomy. In some aspects, the transducer array 112 may include a piezoelectric micromachined ultrasound transducer (PMUT), capacitive micromachined ultrasonic transducer (CMUT), single crystal, lead zirconate titanate (PZT), PZT composite, other suitable transducer types, and/or combinations thereof.

The object 105 may include any anatomy or anatomical feature, such as a kidney, liver, and/or any other anatomy of a subject. The present disclosure can be implemented in the context of any number of anatomical locations and tissue types, including without limitation, organs including the liver, kidneys, gall bladder, pancreas, lungs; ducts; intestines; nervous system structures including the brain, dural sac, spinal cord and peripheral nerves; the urinary tract; as well as valves within the blood vessels, blood, abdominal organs, and/or other systems of the body. In some aspects, the object 105 may include malignancies such as tumors, cysts, lesions, hemorrhages, or blood pools within any part of human anatomy. The anatomy may be a blood vessel, as an artery or a vein of a subject's vascular system, including cardiac vasculature, peripheral vasculature, neural vasculature, renal vasculature, and/or any other suitable lumen inside the body. In addition to natural structures, the present disclosure can be implemented in the context of man-made structures such as, but without limitation, heart valves, stents, shunts, filters, implants and other devices.

The beamformer 114 is coupled to the transducer array 112. The beamformer 114 controls the transducer array 112, for example, for transmission of the ultrasound signals and reception of the ultrasound echo signals. In some aspects, the beamformer 114 may apply a time-delay to signals sent to individual acoustic transducers within an array in the transducer 112 such that an acoustic signal is steered in any suitable direction propagating away from the probe 110. The beamformer 114 may further provide image signals to the processor circuit 116 based on the response of the received ultrasound echo signals. The beamformer 114 may include multiple stages of beamforming. The beamforming can reduce the number of signal lines for coupling to the processor circuit 116. In some aspects, the transducer array 112 in combination with the beamformer 114 may be referred to as an ultrasound imaging component.

The processor 116 is coupled to the beamformer 114. The processor 116 may also be described as a processor circuit, which can include other components in communication with the processor 116, such as a memory, beamformer 114, communication interface 118, and/or other suitable components. The processor 116 may include a central processing unit (CPU), a graphical processing unit (GPU), a digital signal processor (DSP), an application specific integrated circuit (ASIC), a controller, a field programmable gate array (FPGA) device, another hardware device, a firmware device, or any combination thereof configured to perform the operations described herein. The processor 116 may also be implemented as a combination of computing devices, e.g., a combination of a DSP and a microprocessor, a plurality of microprocessors, one or more microprocessors in conjunction with a DSP core, or any other such configuration. The processor 116 is configured to process the beamformed image signals. For example, the processor 116 may perform filtering and/or quadrature demodulation to condition the image signals. The processor 116 and/or 134 can be configured to control the array 112 to obtain ultrasound data associated with the object 105.

The communication interface 118 is coupled to the processor 116. The communication interface 118 may include one or more transmitters, one or more receivers, one or more transceivers, and/or circuitry for transmitting and/or receiving communication signals. The communication interface 118 can include hardware components and/or software components implementing a particular communication protocol suitable for transporting signals over the communication link 120 to the host 130. The communication interface 118 can be referred to as a communication device or a communication interface module.

The communication link 120 may be any suitable communication link. For example, the communication link 120 may be a wired link, such as a universal serial bus (USB) link or an Ethernet link. Alternatively, the communication link 120 may be a wireless link, such as an ultra-wideband (UWB) link, an Institute of Electrical and Electronics Engineers (IEEE) 802.11 Wifi link, or a Bluetooth link.

At the host 130, the communication interface 136 may receive the image signals. The communication interface 136 may be substantially similar to the communication interface 118. The host 130 may be any suitable computing and display device, such as a workstation, a personal computer (PC), a laptop, a tablet, or a mobile phone.

The processor 134 is coupled to the communication interface 136. The processor 134 may also be described as a processor circuit, which can include other components in communication with the processor 134, such as the memory 138, the communication interface 136, an optional speaker 139, and/or other suitable components. The processor 134 may be implemented as a combination of software components and hardware components. The processor 134 may include a central processing unit (CPU), a graphics processing unit (GPU), a digital signal processor (DSP), an application-specific integrated circuit (ASIC), a controller, an FPGA device, another hardware device, a firmware device, or any combination thereof configured to perform the operations described herein. The processor 134 may also be implemented as a combination of computing devices, e.g., a combination of a DSP and a microprocessor, a plurality of microprocessors, one or more microprocessors in conjunction with a DSP core, or any other such configuration. The processor 134 can be configured to generate image data from the image signals received from the probe 110. The processor 134 can apply advanced signal processing and/or image processing techniques to the image signals. In some aspects, the processor 134 can form a three-dimensional (3D) volume image from the image data. In some aspects, the processor 134 can perform real-time processing on the image data to provide a streaming video of ultrasound images of the object 105. In some aspects, the host 130 includes a beamformer. For example, the processor 134 can be part of and/or otherwise in communication with such a beamformer. The beamformer in the in the host 130 can be a system beamformer or a main beamformer (providing one or more subsequent stages of beamforming), while the beamformer 114 is a probe beamformer or micro-beamformer (providing one or more initial stages of beamforming).

The memory 138 is coupled to the processor 134. The memory 138 may be any suitable storage device, such as a cache memory (e.g., a cache memory of the processor 134), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, solid state drives, other forms of volatile and non-volatile memory, or a combination of different types of memory.

The memory 138 can be configured to store subject information, measurements, data, or files relating to a subject's medical history, history of procedures performed, anatomical or biological features, characteristics, or medical conditions associated with a subject, computer readable instructions, such as code, software, or other application, as well as any other suitable information or data. The memory 138 may be located within the host 130. Subject information may include measurements, data, files, other forms of medical history, such as but not limited to ultrasound images, ultrasound videos, and/or any imaging information relating to the subject's anatomy. The subject information may include parameters related to an imaging procedure such as an anatomical scan window, a probe orientation, and/or the subject position during an imaging procedure. The memory 138 can also be configured to store information related to the training and implementation of machine learning algorithms (e.g., neural networks) and/or information related to implementing image recognition algorithms for detecting/segmenting anatomy, image quantification algorithms, and/or image acquisition guidance algorithms, including those described herein.

The display 132 is coupled to the processor circuit 134. The display 132 may be a monitor or any suitable display. The display 132 is configured to display the ultrasound images, image videos, and/or any imaging information of the object 105.

The ultrasound imaging system 100 may be used to assist a sonographer in performing an ultrasound scan. The scan may be performed in a point-of-care setting. In some instances, the host 130 is a console or movable cart. In some instances, the host 130 may be a mobile device, such as a tablet, a mobile phone, or portable computer. During an imaging procedure, the ultrasound system can acquire an ultrasound image of a particular region of interest within a subject's anatomy. The ultrasound imaging system 100 may then analyze the ultrasound image to identify various parameters associated with the acquisition of the image such as the scan window, the probe orientation, the subject position, and/or other parameters. The ultrasound imaging system 100 may then store the image and these associated parameters in the memory 138. At a subsequent imaging procedure, the ultrasound imaging system 100 may retrieve the previously acquired ultrasound image and associated parameters for display to a user which may be used to guide the user of the ultrasound imaging system 100 to use the same or similar parameters in the subsequent imaging procedure, as will be described in more detail hereafter.

In some aspects, the processor 134 may utilize deep learning-based prediction networks to identify parameters of an ultrasound image, including an anatomical scan window, probe orientation, subject position, identify and location of anatomical features, and/or other parameters. In some aspects, the processor 134 may receive metrics or perform various calculations relating to the region of interest imaged or the subject's physiological state during an imaging procedure. These metrics and/or calculations may also be displayed to the sonographer or other user via the display 132.

In some aspects, the host 130 may also include a speaker 180. The speaker 180 may for example be used to provide advisory tones, beeps, or other auditory feedback to the user.

Before continuing, it should be noted that the examples described above are provided for purposes of illustration, and are not intended to be limiting. Other devices and/or device configurations may be utilized to carry out the operations described herein.

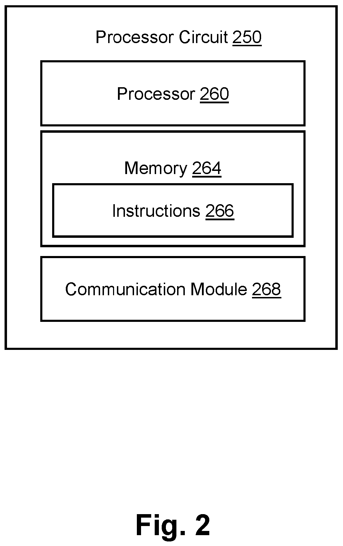

Fig. 2 is a schematic diagram of a processor circuit 250, according to aspects of the present disclosure. The processor circuit 250 may be implemented in the ultrasound imaging system 100, or other devices or workstations (e.g., third-party workstations, network routers, etc.), or on a cloud processor or other remote processing unit, as necessary to implement the method. As shown, the processor circuit 250 may include a processor 260, a memory 264, and a communication module 268. These elements may be in direct or indirect communication with each other, for example via one or more buses.

The processor 260 may include a central processing unit (CPU), a digital signal processor (DSP), a controller, or any combination of general-purpose computing devices, reduced instruction set computing (RISC) devices, application-specific integrated circuits (ASICs), field programmable gate arrays (FPGAs), or other related logic devices, including mechanical and quantum computers. The processor 260 may also comprise another hardware device, a firmware device, or any combination thereof configured to perform the operations described herein. The processor 260 may also be implemented as a combination of computing devices, e.g., a combination of a DSP and a microprocessor, a plurality of microprocessors, one or more microprocessors in conjunction with a DSP core, or any other such configuration.

The memory 264 may include a cache memory (e.g., a cache memory of the processor 260), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an aspect, the memory 264 includes a non-transitory computer-readable medium. The memory 264 may store instructions 266. The instructions 266 may include instructions that, when executed by the processor 260, cause the processor 260 to perform the operations described herein. Instructions 266 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements.

The communication module 268 can include any electronic circuitry and/or logic circuitry to facilitate direct or indirect communication of data between the processor circuit 250, and other processors or devices. In that regard, the communication module 268 can be an input/output (I/O) device. In some instances, the communication module 268 facilitates direct or indirect communication between various elements of the processor circuit 250 and/or the ultrasound imaging system 100. The communication module 268 may communicate within the processor circuit 250 through numerous methods or protocols. Serial communication protocols may include but are not limited to United States Serial Protocol Interface (US SPI), Inter-Integrated Circuit (I²C), Recommended Standard 232 (RS-232), RS-485, Controller Area Network (CAN), Ethernet, Aeronautical Radio, Incorporated 429 (ARINC 429), MODBUS, Military Standard 1553 (MIL-STD-1553), or any other suitable method or protocol. Parallel protocols include but are not limited to Industry Standard Architecture (ISA), Advanced Technology Attachment (ATA), Small Computer System Interface (SCSI), Peripheral Component Interconnect (PCI), Institute of Electrical and Electronics Engineers 488 (IEEE-488), IEEE-1284, and other suitable protocols. Where appropriate, serial and parallel communications may be bridged by a Universal Asynchronous Receiver Transmitter (UART), Universal Synchronous Receiver Transmitter (USART), or other appropriate subsystem.

External communication (including but not limited to software updates, firmware updates, model sharing between the processor and central server, or readings from the ultrasound imaging system 100) may be accomplished using any suitable wireless or wired communication technology, such as a cable interface such as a universal serial bus (USB), micro USB, Lightning, or FireWire interface, Bluetooth, Wi-Fi, ZigBee, Li-Fi, or cellular data connections such as 2G/GSM (global system for mobiles) , 3G/UMTS (universal mobile telecommunications system), 4G, long term evolution (LTE), WiMax, or 5G. For example, a Bluetooth Low Energy (BLE) radio can be used to establish connectivity with a cloud service, for transmission of data, and for receipt of software patches. The controller may be configured to communicate with a remote server, or a local device such as a laptop, tablet, or handheld device, or may include a display capable of showing status variables and other information. Information may also be transferred on physical media such as a USB flash drive or memory stick.

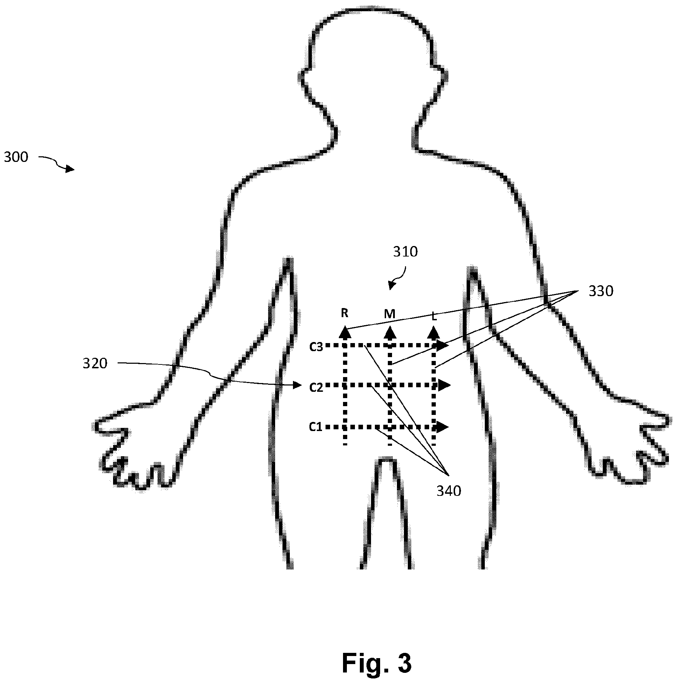

Fig. 3 is a schematic, diagrammatic representation of a patient 300, according to aspects of the present disclosure. Visible on the abdomen 310 of the patient 300 is a desired sweep pattern 320 intended to capture images of desired features of the patient's anatomy. The sweep pattern 320 includes multiple vertical sweep lines 330 and multiple horizontal sweep lines 340. Each sweep line 330, 340 represents a desired path for one imaging sweep of the abdomen 310. In the example shown in Fig. 3, the sweep pattern includes three vertical sweep lines 330 labeled L (patient's left), M (patient's middle), and R (patient's right), all in an upward direction with respect to the patient, and three horizontal sweep lines 340 labeled C1 (bottom), C2 (middle), and C3 (top), all in a right-to-left direction with respect to the patient. However, it is understood that a sweep pattern 320 may include more or fewer sweep lines 330, including vertical sweep lines 330, horizontal sweep lines 330, or combinations thereof, in any combination of upward, downward, left, or right directions, based on the patient's fundal height. For example, if the patient's belly is bigger in size, more sweeps may be needed. Furthermore, a sweep pattern 320 may cover other portions of the patient's body, including but not limited to the head, neck, spine, limbs, etc. Examples of blind sweep protocol include but are not limited to obstetric sweep imaging (OSI), volume sweep imaging (VSI), 6-Stage, Fetal Age Machine Learning Initiative (FAMLI), and Philips.

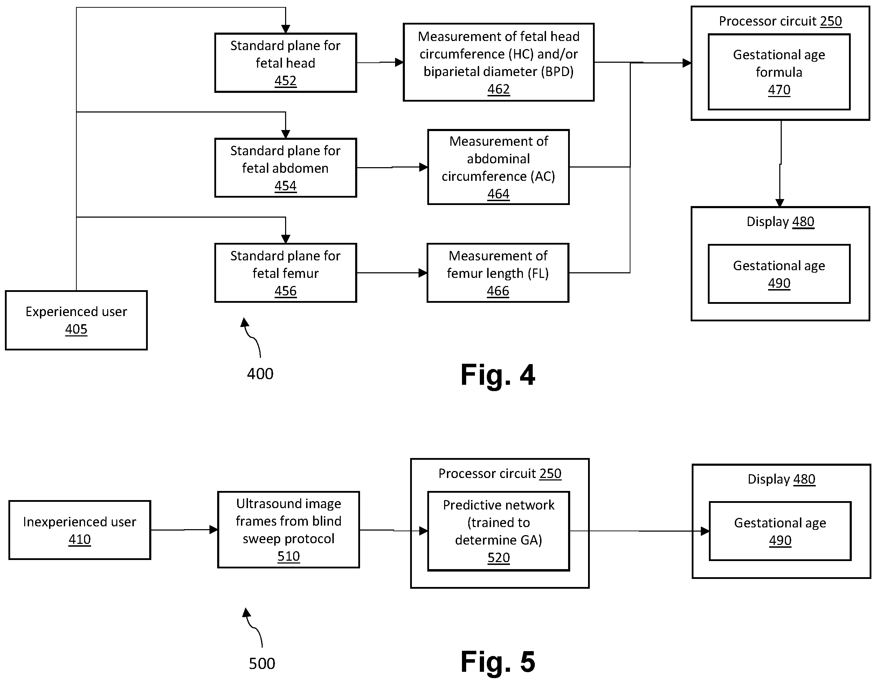

These sweep patterns represent desired probe motion information, including desired positions, a desired velocity or velocities, and/or a desired orientation of the ultrasound probe while the ultrasound probe is obtaining a plurality of ultrasound image frames during the sweep. It is noted that the desired sweep patterns or blind sweep protocols stored in a memory of the processor may include only vertical sweeps, only horizontal sweeps, may include a grid (e.g., 3x3, 5x5, etc.) of vertical and horizontal sweeps, and may also include associated parameters such as desired probe motion (e.g., positions, velocities, and/or orientations) stored in the memory (e.g., blind sweep protocol 440 in Fig. 5). Depending on the implementation, sweeps may include curved, diagonal, and other types of sweeps. The protocols and their associated parameters can for example be based on standards established by authorities in the field (physician organizations, sonographer organizations, etc.), published in scholarly journals/textbooks, etc.

Fig. 4 is a schematic, diagrammatic representation, in hybrid flow diagram / block diagram form, of an example present-day GA measurement workflow for an experienced user 405, according to aspects of the present disclosure. Using the ultrasound probe, the experienced user 405 (e.g., a clinician or sonographer) finds the standard plane 452 for fetal head measurement, and uses that image to perform a fetal head measurement 462 (e.g., a measurement of the head circumference (HC) and/or a measurement of the biparietal diameter (BPD)). Similarly, the experienced user 405 finds the standard plane 454 for fetal abdomen measurement, and uses that image to perform a measurement 464 of the fetal abdominal circumference (AC). The experienced user 405 also finds the standard plane 456 for fetal femur measurement, and uses that image to perform a measurement 466 of the fetal femur length. The head measurement 462, abdominal measurement 464, and/or femur measurement 466 are then received by the processor circuit 250 and used in a gestational age (GA) formula 470, which uses the available information to estimate gestational age 490, which may then be shown on a display 480. This workflow 400 relies on significant expertise on the part of the user, both to find the standard measurement planes and to perform the standard measurements. Thus, inexperienced users will generally not be able to perform this workflow, making it unsuitable for many under-resourced settings where highly trained personnel may not be available. The standard plane 452 can be a pre-defined or pre-determined plane that is determined by authorities in the field (physician organizations, sonographer organizations, etc.) and published in scholarly journals/textbooks, etc.

Fig. 5 is a schematic, diagrammatic representation, in hybrid flow diagram / block diagram form, of an example present-day GA measurement workflow for an inexperienced user 410, according to aspects of the present disclosure. Using the ultrasound probe, the inexperienced user 410 acquires ultrasound image frames 510 using a blind sweep protocol, which are stored in the processor circuit 250 and used by a predictive network 520 that has been trained to determine gestational age 490, which may then be shown on a display 480. Because the image frames 510 from the blind sweep protocol are unlikely to contain the standard measurement planes 452, 454, 456 (see Fig. 4), the GA prediction 490 may have low accuracy or low confidence, and may not be seen as reliable by the user 410 or by trained clinicians 405. Thus, a need exists for improved workflows that can be performed by the inexperienced user 410, that produce higher-confidence results.

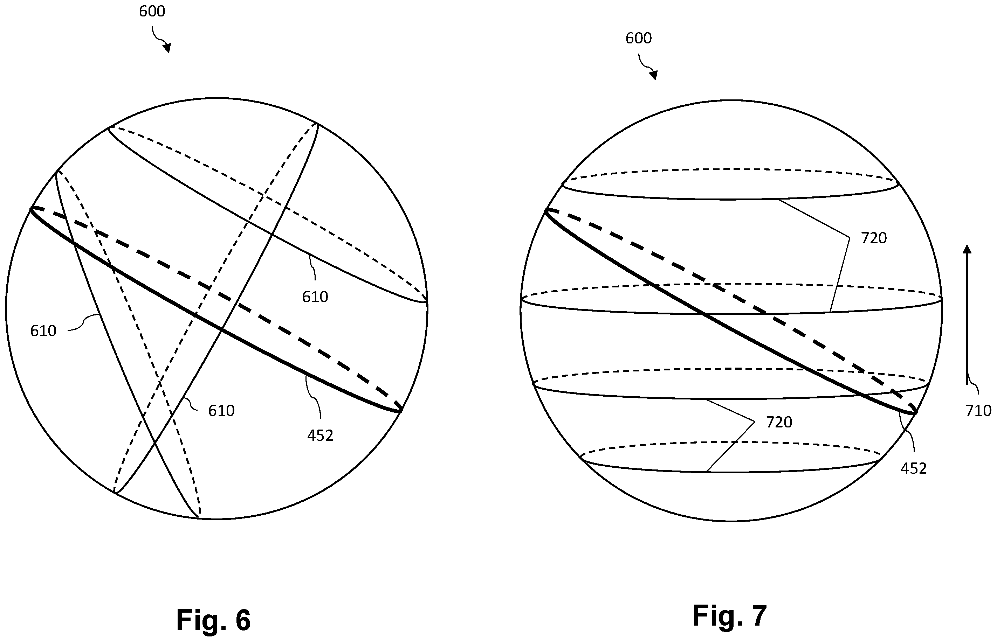

Fig. 6 is a schematic, diagrammatic representation of a fetal head 610, according to aspects of the present disclosure. Visible is the standard plane 452 for fetal head measurement. The standard plane 452 may for example contain certain landmark anatomy such as: transverse view of the fetal head at the level of the thalami; ideal angle of insonation roughly 90∘ to the midline echoes; symmetrical appearance of both hemispheres; midline echo (falx cerebri) interrupted anteriorly only by the cavum septi pellucidi; and cerebellum not visible. These features make the standard plane 452 identifiable, and provide a consistent way of measuring the head across different patients and across different visits for the same patient. Other, non-standard imaging planes 610 are at incorrect angles and/or latitudes, and are thus likely to deliver incorrect measurements of the head circumference and/or head diameter.

Fig. 7 is a schematic, diagrammatic representation of a fetal head 610, according to aspects of the present disclosure. Visible is the standard plane 452 for fetal head measurement. A blind sweep 710 captures images at multiple planes 720 that are usually parallel to each other (because the longitudinal nature of the sweep, the straight path of motion of the transducer takes, as shown in Fig. 3). The multiple planes 720 each show a different diameter and circumference and are each at a different angle compared the standard plane 452. Because the fetus could be at any orientation relative to the mother's abdomen, any given blind sweep 710 is unlikely to align with, and thus correctly image, the standard measurement plane 452.

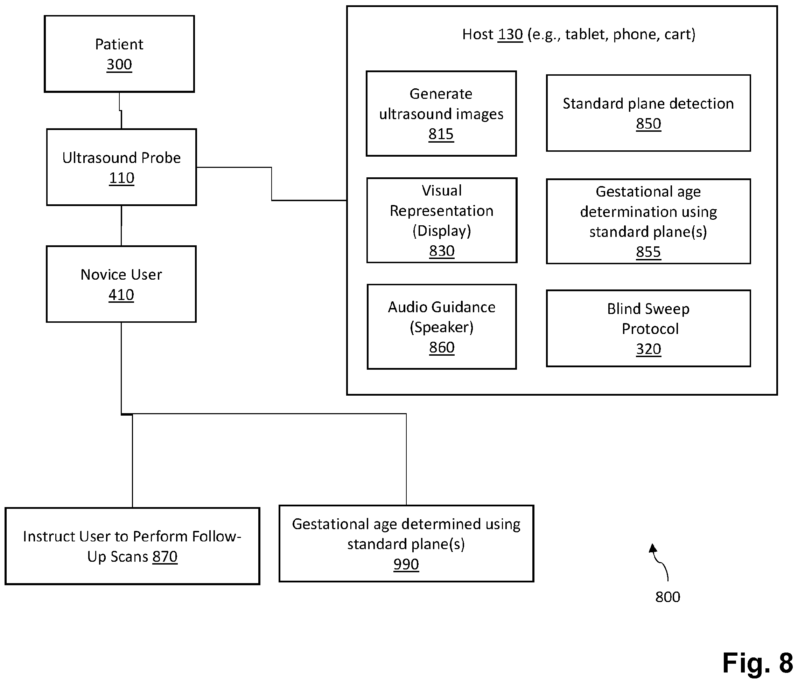

Fig. 8 is a schematic, diagrammatic representation, in hybrid block diagram/flow diagram form, of an example ultrasound sweep standard plane detection system 800, according to aspects of the present disclosure. An ultrasound probe 110 operated by a novice user 410 performs a blind sweep protocol 320 on the body of a patient 300 and sends ultrasound imaging data to a host 130, such as a tablet, smartphone, ultrasound cart, etc. In step 815, the host 130 generates ultrasound images using the ultrasound image data obtained by the ultrasound probe 110. The host 130 can control the ultrasound probe 110 to obtain the ultrasound image data (e.g., the host 130 establishes communication with the ultrasound probe 110, the host 130 sends control signals to start and/or stop acquisition of ultrasound image data, the host 130 sends power signals to power the ultrasound probe 110, etc.).

The host 130 may then generate a visual representation 830 or audio guidance 860 indicating where and how the user should re-scan 870 the mother's abdomen in order to capture one or more standard measurement planes for the fetus. While these re-scans are being performed, the host 130 performs standard plane detection 850 (e.g., with an anatomy detector or classifier such as a deep learning network), and uses the anatomical images in the standard planes to perform gestational age determination 855, in order to determine (and report) the gestational age 890.

Flow diagrams and block diagrams are provided herein for exemplary purposes; a person of ordinary skill in the art will recognize myriad variations that nonetheless fall within the scope of the present disclosure. For example, any of the steps described herein may optionally include an output to a user of information relevant to the step, and may thus represent an improvement in the user interface over existing art by providing information not otherwise available. Similarly, block diagrams may show a particular arrangement of components, modules, services, steps, processes, or layers, resulting in a particular data flow. It is understood that some aspects of the systems disclosed herein may include additional components, that some components shown may be absent from some aspects, and that the arrangement of components may be different than shown, resulting in different data flows while still performing the methods described herein. The logic of flow diagrams may be shown as sequential. However, similar logic could be parallel, massively parallel, object oriented, real-time, event-driven, cellular automaton, or otherwise, while accomplishing the same or similar functions. In order to perform the methods described herein, a processor may divide each of the steps described herein into a plurality of machine instructions, and may execute these instructions at the rate of several hundred, several thousand, several million, or several billion per second, in a single processor or across a plurality of processors. Such rapid execution may be necessary in order to execute the method in real time or near-real time as described herein.

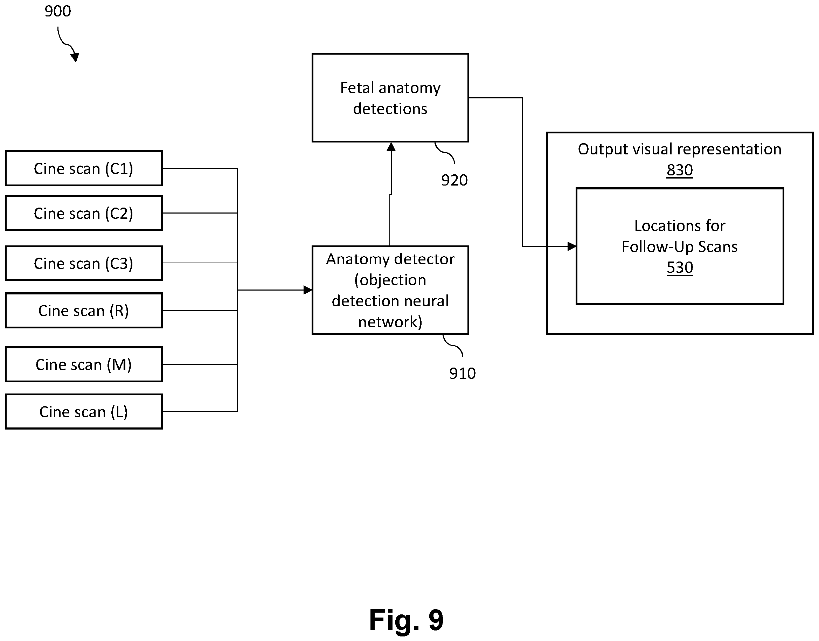

Fig. 9 is a schematic, diagrammatic representation, in hybrid block diagram/flow diagram form, of an example ultrasound sweep anatomy detection method 900 to determine fetal gestational age, according to aspects of the present disclosure. Images from a sweep of the blind sweep protocol can be organized into an image sequence known as a cine scan or cineloop (e.g., multiple image frames making up a video segment). The host thus receives or assembles cine scans C1, C2, C3, R, M, and L, corresponding to the blind abdominal sweeps C1, C2, C3, R, M, and L shown in Fig. 3.

In step 910, these cine scans are fed into an object detector or anatomy detector (e.g., a trained neural network or deep learning network) to detect the presence of and, if present, the location of the anatomical region of interest (e.g., head, abdomen, femur, etc.) in each ultrasound image frame of the cine scan.

The resulting fetal anatomy detections 920 are then represented in an output visual representation 830 showing the locations 530 (and possibly probe orientations) for follow-up scans to be performed by the user, in order to capture the standard imaging planes.

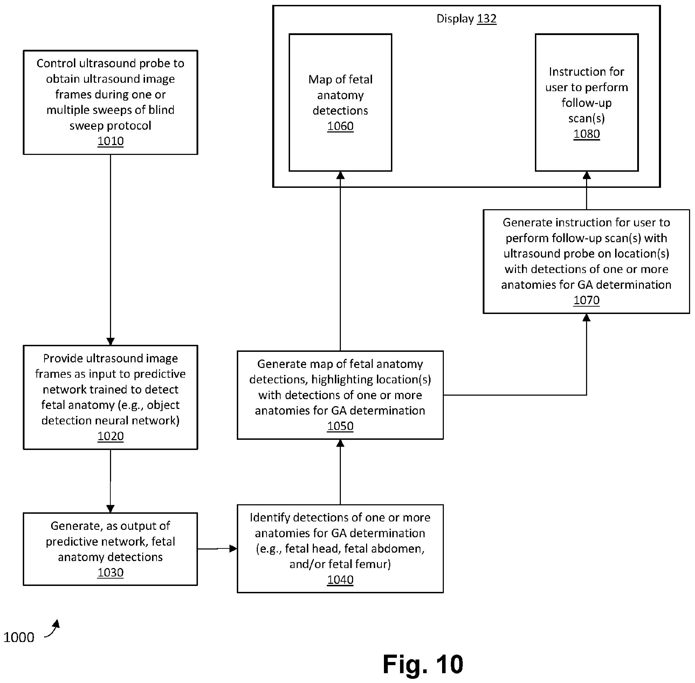

Fig. 10 is a schematic, diagrammatic representation, in flow diagram form, of an example standard plane imaging method 1000, according to aspects of the present disclosure.

In step 1010, the method 1000 includes controlling the ultrasound probe to obtain ultrasound image frames during one or more sweeps of the blind sweep protocol. Execution then proceeds to step 1020.

In step 1020, the method 1000 includes providing the ultrasound mage frames as inputs to a predictive network trained to detect fetal anatomy (e.g., an object detection neural network or deep learning network). Execution then proceeds to step 1030.

In step 1030, the method 1000 includes generating, as an output of the predictive network, the fetal anatomy detections. Execution then proceeds to step 1040.

In step 1040, the method 1000 includes identifying detections of one or more anatomies that are used for GA determination (e.g., the fetal head, fetal abdomen, fetal femur, etc.). Execution then proceeds to step 1050.

In step 1050, the method 1000 includes generating a map 1060 of the fetal anatomy detections that can be shown on the display 132, and that highlights the locations with the detections of the one or more anatomies used for GA determination. Execution then proceeds to step 1070.

In step 1070, the method 1000 includes generating an instruction 1080 on the display 132 for the user to perform one or more follow-up scans with the ultrasound probe on the locations where the detections were identified, of the fetal anatomies used for GA determination. If the instructions are followed correctly, the re-scans will include the standard measurement planes for the target anatomies. The method 1000 is now complete.

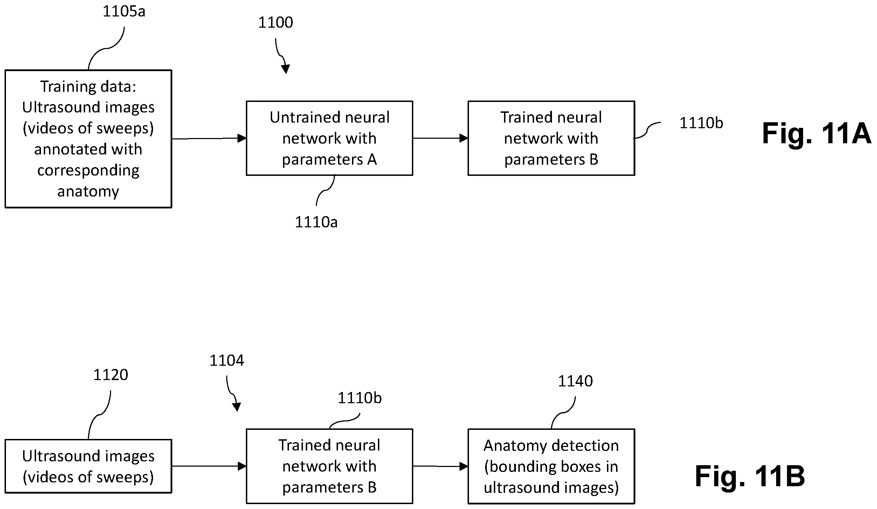

Fig. 11A is a schematic, diagrammatic overview, in block diagram form, of a training mode 1100 for an untrained neural network 1110a, according to aspects of the present disclosure. In the example shown in Fig. 11A, a set of training data 1105a includes ultrasound cineloops of probe sweeps annotated with the corresponding anatomy (fetal head, abdomen, femur, etc.). The training data 1105a is fed into an untrained neural network 1110a in an iterative training process that will be familiar to a person of ordinary skill in the art.

The parameters of a network model (e.g., the weights at each artificial neuron) are initialized with initial values A that may be random values or with results from training on prior datasets. In an iterative process, the network is used to make detection inferences on the training images, the results are compared with the ground truth annotations, and an optimizer is used to adjust the network parameters B until a metric of accuracy is maximized.

Thus, an output of this training process 1100 is a trained neural network 1110b, wherein the parameters B (e.g., weights) are optimized for generating accurate bounding boxes for the anatomy imaged in the training data 1105a.

Fig. 11B is a schematic, diagrammatic overview, in block diagram form, of an inference mode or clinical usage mode 1104 for the trained neural network 1110b, according to aspects of the present disclosure. In clinical usage, an ultrasound video, cineloop, or cine sweep 1120 of the blind sweep is fed to the trained and validated neural network 1110b for analysis. The trained and validated neural network 1110b then produces, as an output, anatomy detection bounding boxes 1140 for each image (or the entire sweep). In some aspects, a confidence value can be determined as a normalized value in the range [0-1], where 0 indicates lowest confidence, and 1 indicates highest confidence that the detection is correct.

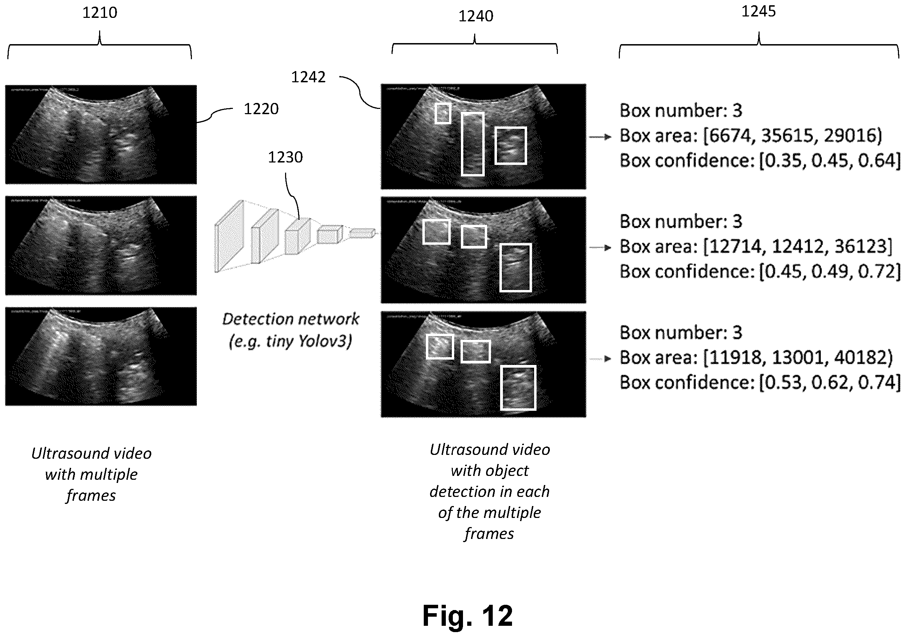

Fig. 12 is a schematic, diagrammatic illustration, in block diagram form, of the detection of anatomy (e.g., the fetal head, abdomen, femur, etc.), according to aspects of the present disclosure. A cineloop 1210 comprising multiple frames 1220 is fed into a trained object detector 1230.

The object detector 1230 may implement or include any suitable type of learning network. For example, in some aspects, the object detector 1230 could include a neural network, such as a convolutional neural network (CNN). In addition, the convolutional neural network may additionally or alternatively be an encoder-decoder type network, or may utilize a backbone architecture based on other types of neural networks, such as an object detection network, classification network, etc. One example backbone network is the Darknet YOLO backbone, (e.g., Yolov3) which can be used for object detection. The CNN may for example include a set of N convolutional layers, where N may be any positive integer. Fully connected layers can be omitted when the CNN is a backbone. The CNN may also include max pooling layers and/or activation layers. Each convolutional layer may include a set of filters configured to extract features from an input (e.g., from a frame of the ultrasound video). The value N and the size of the filters may vary depending on the aspects. In some instances, the convolutional layers may utilize any non-linear activation function, such as for example a leaky rectified non-linear (ReLU) activation function and/or batch normalization. The max pooling layers gradually shrink the high-dimensional output to a dimension of the desired result (e.g., bounding boxes of a detected feature). Outputs of detection network may include numerous bounding boxes, with most having very low confidence scores and thus being filtered out or ignored. Fully connected layers may be referred to as perception or perceptive layers. In some aspects, perception/perceptive and/or fully connected layers may be found in object detector 1230 (e.g., a multi-layer perceptron).

These descriptions are included for exemplary purposes; a person of ordinary skill in the art will appreciate that other types of learning models, with features similar to or dissimilar to those described above, may be used instead or in addition, without departing from the spirit of the present disclosure.

Outputs of the object detector 1230 may include an annotated cineloop 1240 made up of a plurality of annotated image frames 1242, possibly including per-frame metrics 1245 such as the confidence level of the detections.

The systems and methods disclosed herein are broadly applicable to different types of features, and can for example draw boxes around the placenta, amniotic fluid, cervix, fetus, or other anatomical features depending on the implementation. The object detector can be one class or multi-class, depending how the model is built. If another detector is trained separately, then both models can be run separately (e.g., one model for each feature type). Otherwise, multiple feature classes can be identified, and enclosed in detection boxes, at the same time. The ML model for placenta detection can use exactly the same structure as a model for cervix detection. One can either train/run a single detector that detects multiple feature types (a "multi-class detector") and provides their locations as an output, along with the confidence score and feature type (class) of each detection. Alternatively, one could run several "single-class" detectors, each trained to detect a single feature type/class. These separate single-class detectors may have the same architecture (e.g., layers and connections), but would have been trained with different data (e.g., different images and/or annotations).

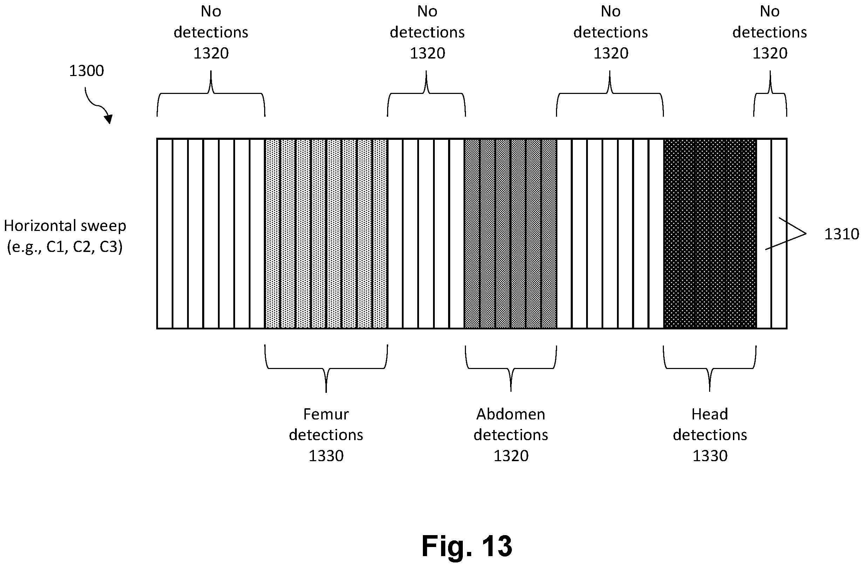

Fig. 13 is a schematic, diagrammatic representation of the ultrasound image frames 1310 of a horizontal blind sweep 1300 (e.g., a C1, C2, or C3 sweep), according to aspects of the present disclosure. Some frames 1310 contain no detections 1320. Other frames 1310 contain femur detections 1330, abdomen detections 1320, and head detections 1330, which identify locations that can be rescanned in order to capture the corresponding standard measurement planes. Although the example of Fig. 13 shows a horizontal sweep, similar detection steps are performed for a vertical sweep.

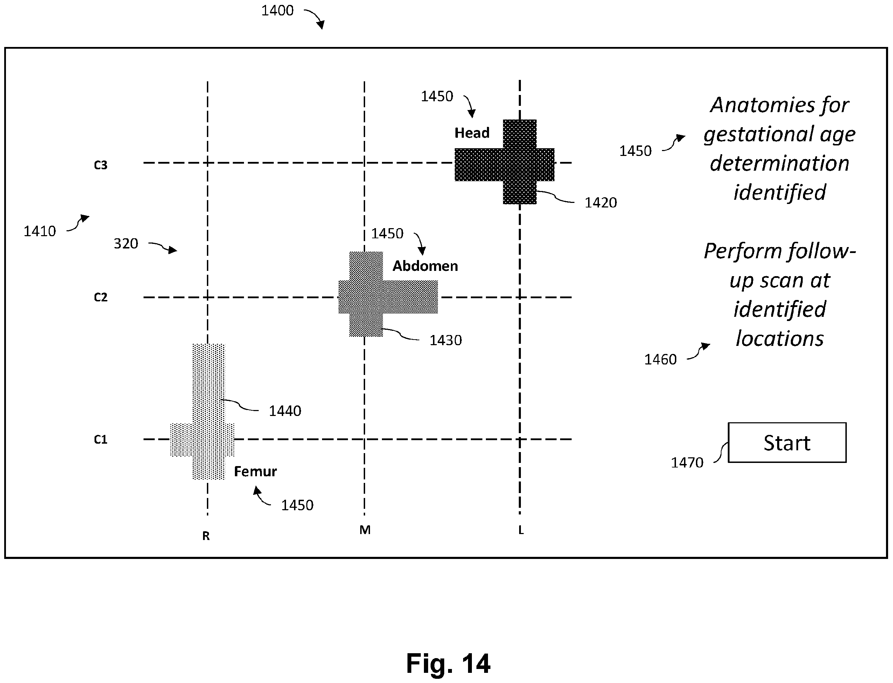

Fig. 14 is a screen display 1400 of an example ultrasound sweep standard plane detection system, according to aspects of the present disclosure. The screen display 1400 includes an anatomy map 1410 showing the blind sweep protocol 320 overlaid with the locations of the fetal head detections 1420, fetal abdomen detections 1430, and fetal femur detections 1440. There are also informative text labels 1450, as well as instructions 1460 for the user to perform the follow-up scans at the identified locations. A start button 1470 allows the user to indicate to the system when they are ready to begin the follow-up scans.

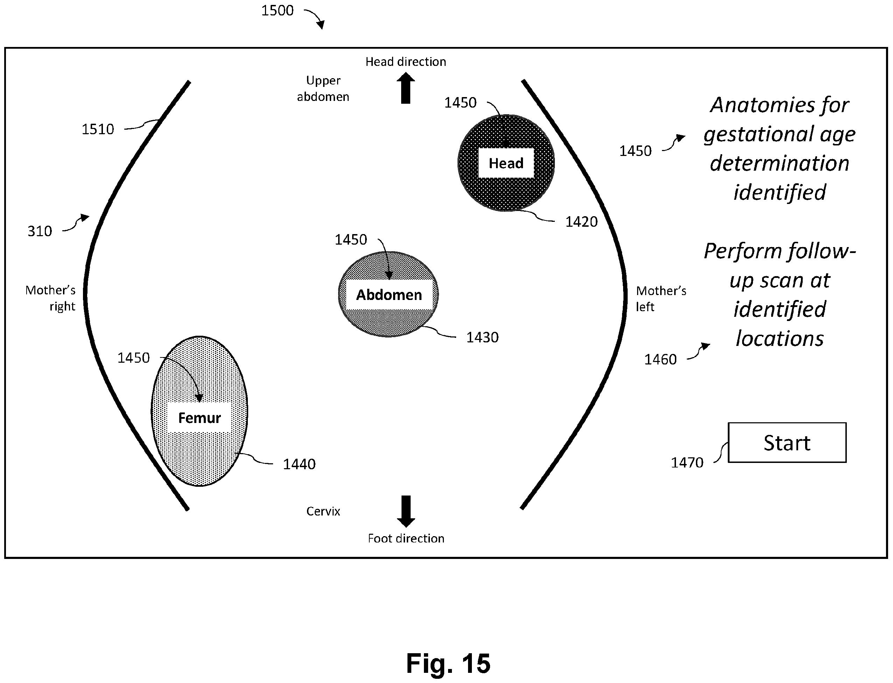

Fig. 15 is a screen display 1500 of an example ultrasound sweep standard plane detection system, according to aspects of the present disclosure. The screen display 1500 includes an anatomy map 1510 showing the mother's abdomen 310 overlaid with the locations of the fetal head detections 1420, fetal abdomen detections 1430, and fetal femur detections 1440. There are also informative text labels 1450, as well as instructions 1460 for the user to perform the follow-up scans at the identified locations. A start button 1470 allows the user to indicate to the system when they are ready to begin the follow-up scans.

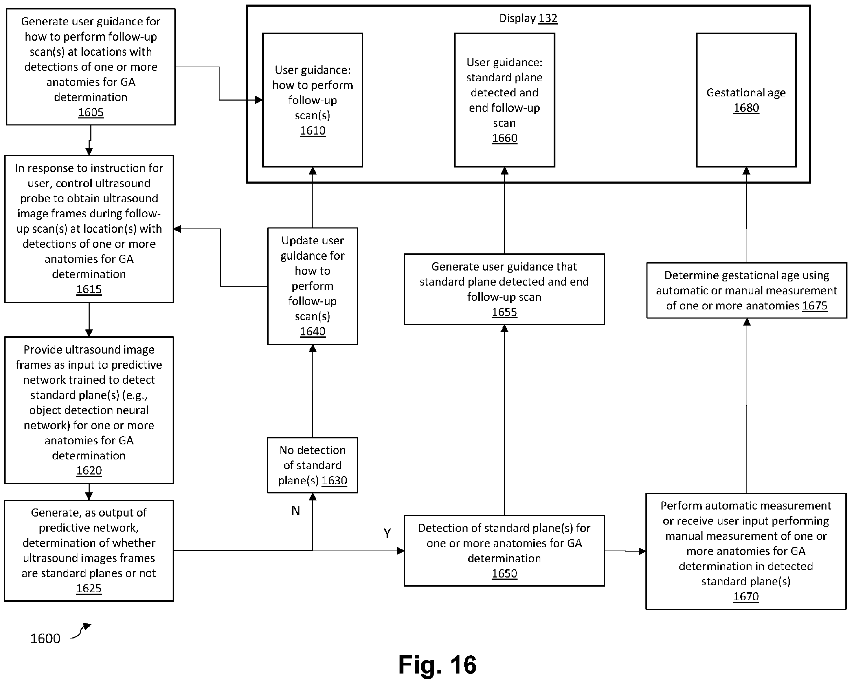

Fig. 16 is a schematic, diagrammatic representation, in flow diagram form, of an example ultrasound sweep standard plane detection method 1600, according to aspects of the present disclosure.

In step 1605, the method 1600 includes generating user guidance 1610 for how to perform the follow-up scan(s) at the locations where the anatomies were detected that are useful for GA determination (e.g., fetal head, abdomen, and femur). This user guidance 1610 is then shown on the display 132. User guidance may for example include sliding the probe, rocking the probe, sweeping the probe, fanning the probe, increasing or decreasing pressure on the probe, rotating the probe, and otherwise. Execution then proceeds to step 1615.

In step 1615, the method 1600 includes, in response to an instruction from the user (e.g., pressing the start button), controlling the ultrasound probe to obtain ultrasound image frames during the follow-up scan(s) at the locations where the GA determination anatomies were detected. Execution then proceeds to step 1620.

In step 1620, the method 1600 includes providing the ultrasound image frames as inputs to a predictive network trained to detect standard planes for one or more anatomies useful for GA determination. One example of the predictive network trained to detect standard planes is an anatomy detector or object detection neural network. Other examples include a You Only Look Once (YOLO) network, Region-based Convolutional Neural Network (R-CNN), selective search algorithm, Histogram of Oriented Gradients (HOG) based algorithm, multimodal Large Language Model (LLM) based neural network, or edge detection algorithms or feature extraction algorithms all which are trained to work for detecting standard planes. Execution then proceeds to step 1625.

In step 1625, the method 1600 includes generating, as an output of the predictive network, a determination of whether the ultrasound image frames are standard planes or not. If yes, execution proceeds to step 1650. If no, execution proceeds to step 1630.

In step 1630, the method 1600 includes determining that there has been no detection of standard planes. Execution then proceeds to step 1640.

In step 1640, the method 1600 includes updating and displaying the user guidance 1610 for how to perform the follow-up scan(s). For example, predictive networks (e.g., neural networks), such as those using reinforcement learning, trained to generate user guidance on different position(s) and/or orientation(s) to move the ultrasound probe can be used. Examples include those described in U.S. Patent No. 12,048,589, titled "Guided ultrasound imaging", U.S. Publication No. 2021/0369249, titled "Deep learning-based ultrasound imaging guidance and associated devices, systems, and methods", U.S. Publication No. 2021/0000446, titled "Ultrasound imaging plane alignment guidance for neural networks and associated devices, systems, and methods", U.S. Publication No. 2020/0352542, titled "Guided-transcranial ultrasound imaging using neural networks and associated devices, systems, and methods", and International Publication No. WO2024223599A1, titled "Guided cardiac ultrasound imaging to minimize apical foreshortening", each of which is incorporated by reference herein in its entirety. Execution then returns to step 1615.

In step 1650, the method 1600 includes performing detection of one or more standard planes, for one or more anatomies used for GA determination. Execution then proceeds to steps 1655 and 1670.

In step 1655, the method 1600 includes generating user guidance 1660 that the standard plane(s) have been detected and to end the follow-up scan(s). This user guidance 1660 can then be shown on the display 132.

In step 1670, the method 1600 includes performing an automatic measurement, or receiving a user input performing a manual measurement, of one or more anatomies used for GA determination in the detected standard planes. Automatic measurement is described for example in International Publication No. WO2024104857A1, filed November 8, 2023, entitled "Automatic measurement point detection for anatomy measurement in anatomical images", incorporated by reference as though fully set forth herein. Execution then proceeds to step 1675.

In step 1675, the method 1600 includes determining the gestational age 1680 using the automatic or manual measurement of the one or more anatomies. The gestational age 1680 can then be shown on the display 132. The method 1600 is now complete.

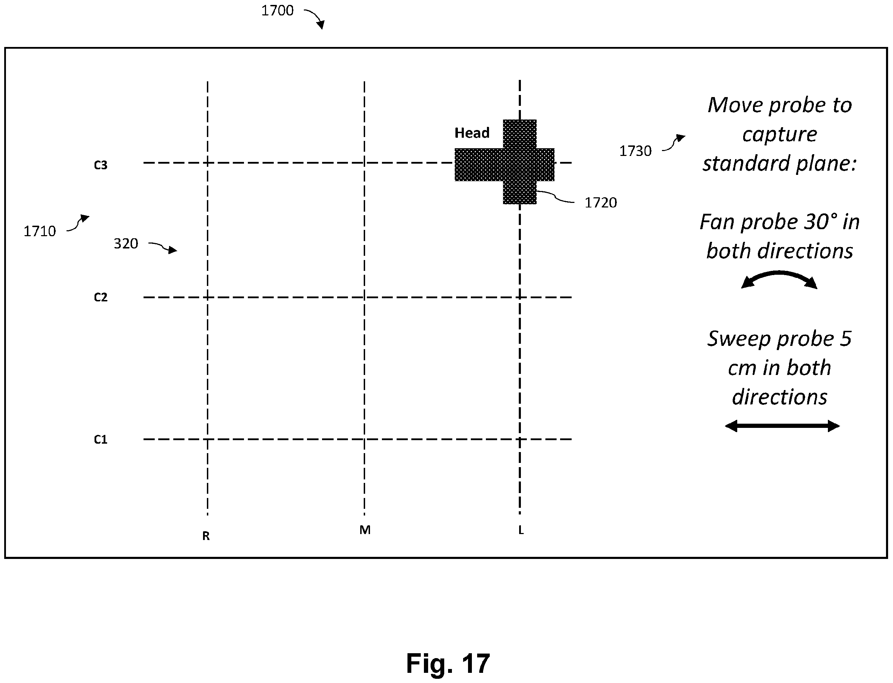

Fig. 17 is a screen display 1700 of an example ultrasound sweep standard plane detection system, according to aspects of the present disclosure. The screen display 1700 includes an anatomy map 1710 showing the blind sweep protocol 320 overlaid with the location 1720 where a re-scan is needed. Also visible are user instructions 1730, which may include sliding the probe, rocking the probe, sweeping the probe, fanning the probe, increasing or decreasing pressure on the probe, rotating the probe, and otherwise moving the probe at the location 1720, in order to capture an ultrasound image of the corresponding standard measurement plane. In the example shown In Fig. 17, this would be the standard head measurement plane.

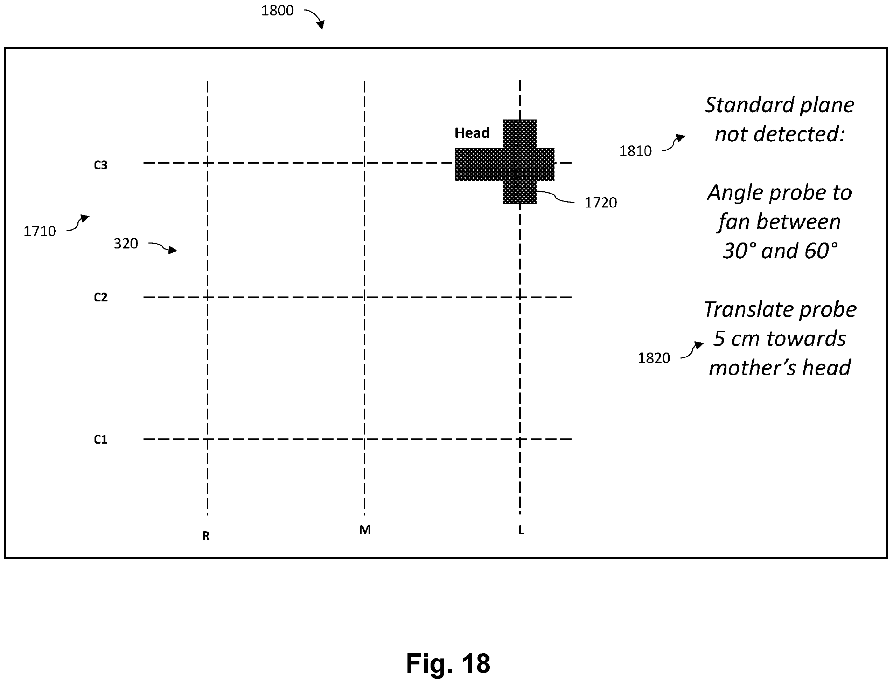

Fig. 18 is a screen display 1800 of an example ultrasound sweep standard plane detection system, according to aspects of the present disclosure. The screen display 1800 includes an anatomy map 1710 showing the blind sweep protocol 320 overlaid with the location 1720 where the re-scan is needed. In the case of Fig. 18, the re-scan has not been successful in imaging one of the standard measurement planes, so a "not detected" message 1810 is displayed, along with updated instructions 1820 of probe movements for the user to perform at the location 1720. The purpose of the updated instructions is to obtain the desired image of the standard imaging plane, which can be used for gestational age measurement.

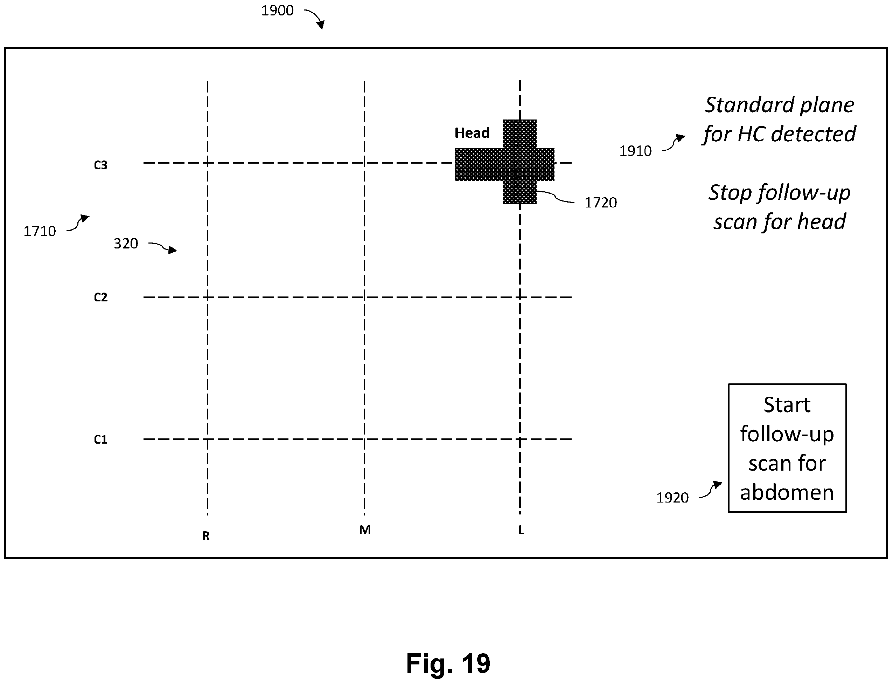

Fig. 19 is a screen display 1900 of an example ultrasound sweep standard plane detection system, according to aspects of the present disclosure. The screen display 1900 includes an anatomy map 1710 showing the blind sweep protocol 320 overlaid with the location 1720 where the re-scan is needed. In the example shown in Fig. 19, the re-scan has successfully imaged the standard measurement plane for the desired anatomy (in this case, the fetal head). Thus, a message 1910 is displayed to the user, indicating that the standard plane has been detected and the user can stop scanning. Also visible is a start button 1920 which, when pressed, begins the follow-up scan for the next GA determination anatomy (in this case, the fetal abdomen).

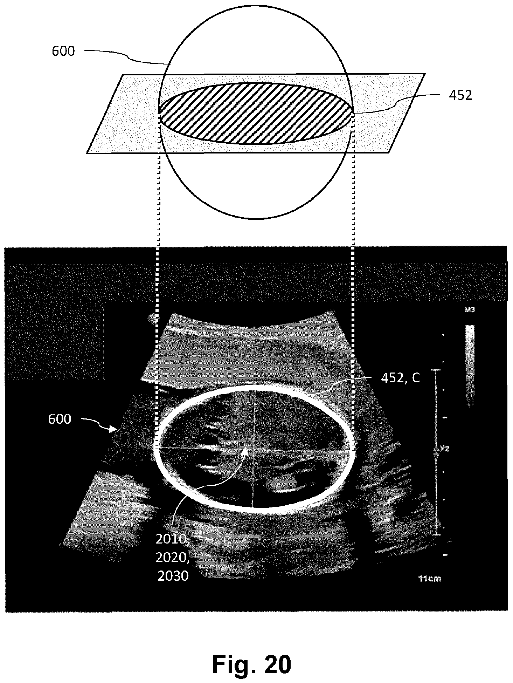

Fig. 20 is a schematic, diagrammatic representation of the standard measurement plane 452 of a fetal head 600, according to aspects of the present disclosure. Depending on the implementation, the standard measurement plane 452 may be detected by the simultaneous presence of a first anatomy/anatomical feature 2010 (e.g., the thalami;), a second anatomy/anatomical feature 2020 (e.g., symmetrical appearance of both hemispheres), and a third anatomy/anatomical feature 2030 (e.g., the cavum septi pellucid), which would not be simultaneously visible in an imaging plane other than the standard measurement plane. In some cases, an object detector or other deep learning network may detect the standard measurement plane directly (e.g., as a single object). In other cases, the object detector or other deep learning network may detect the individual anatomies/anatomical features 2010, 2020, 2030, or confirm the absence of other anatomies (e.g., the cerebellum) to deduce or infer the detection of the standard measurement plane. Once the standard measurement plane is detected, the system (or the user) can measure the head circumference C in that plane. This head circumference can then be used directly to compute the gestational age, or may be used as an input to a GA formula that includes other measurements such as femur length and/or abdominal circumference or diameter.

Although the detection process is shown here for the fetal head, it is understood that a similar process can be followed with the fetal abdomen and fetal femur, in order to obtain images of the standard measurement planes (and thus, measurements) for these anatomies as well.

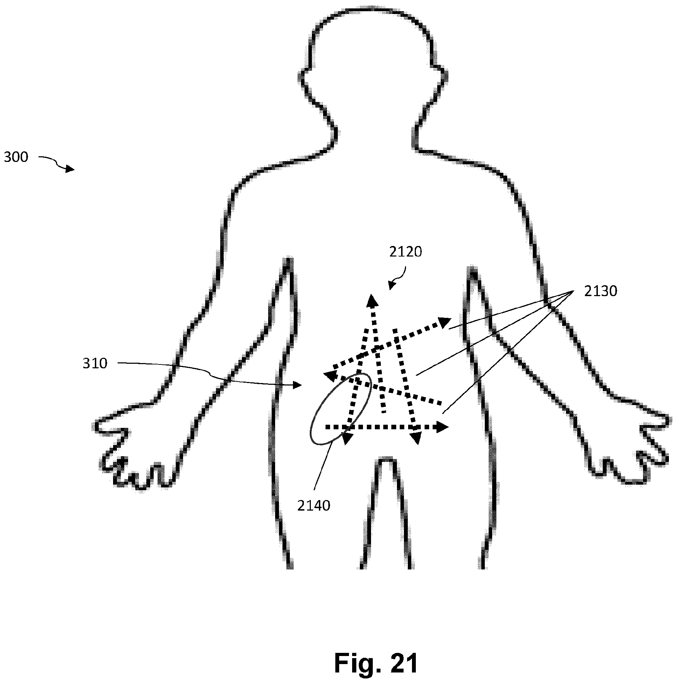

Fig. 21 is a schematic, diagrammatic representation of a patient 300, according to aspects of the present disclosure. Visible on the abdomen 310 of the patient 300 is a random sweep pattern 2120 intended to capture images of one or more standard measurement planes 2140 of the patient's anatomy. The sweep pattern 2120 includes multiple random sweep lines 2130. Each sweep line 2130 may be at a different direction, speed, pressure, fan angle, or rotation angle than the other sweep lines 2130, in order to maximize coverage of the abdomen, and thus maximize the chance that one or more of the sweep lines 2130 will coincide with a standard measurement plane for the fetal head, fetal abdomen, fetal femur, or other anatomy that may be useful in estimating gestational age.

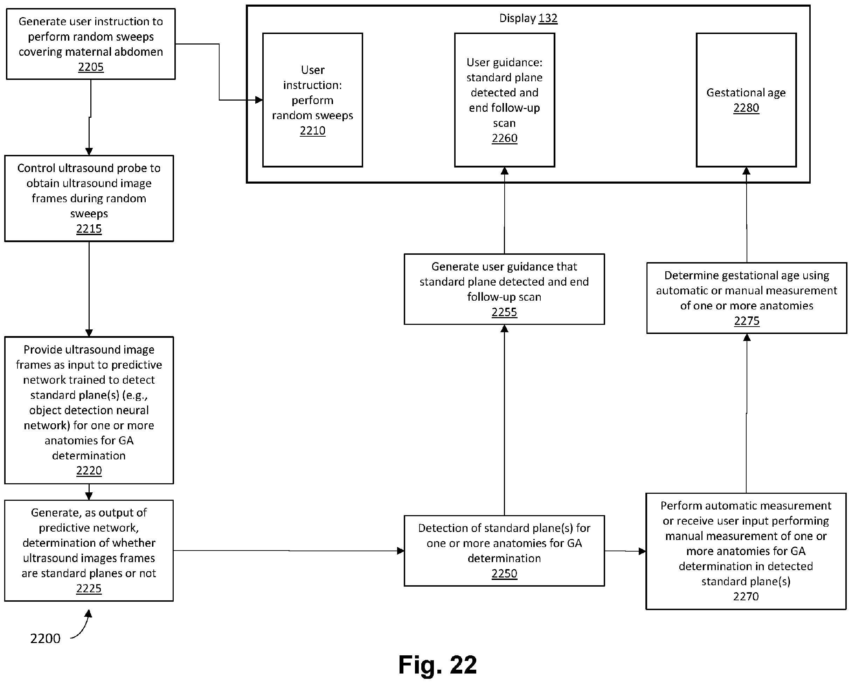

Fig. 22 is a schematic, diagrammatic representation, in flow diagram form, of an example ultrasound sweep standard plane detection method 2200, according to aspects of the present disclosure.

In step 2205, the method 2200 includes generating user guidance 2210 to perform random sweeps across the patient's abdomen. This user guidance 2210 is then shown on the display 132. Random sweeps may for example include sliding the probe, rocking the probe, sweeping the probe, fanning the probe, increasing or decreasing pressure on the probe, rotating the probe, and otherwise. Execution then proceeds to step 2215.

In step 2215, the method 2200 includes, in response to an instruction from the user (e.g., pressing the start button), controlling the ultrasound probe to obtain ultrasound image frames during the random sweeps. Execution then proceeds to step 2220.

In step 2220, the method 2200 includes providing the ultrasound image frames as inputs to a predictive network trained to detect standard planes for one or more anatomies useful for GA determination. One example of the predictive network trained to detect standard planes is an anatomy detector or object detection neural network, as described above. Execution then proceeds to step 2225.

In step 2225, the method 2200 includes generating, as an output of the predictive network, a determination of whether the ultrasound image frames are standard planes or not. If yes, execution proceeds to step 2250. If no, execution returns to step 2225.

In step 2250, the method 2200 includes performing detection of one or more standard planes, for one or more anatomies used for GA determination. Execution then proceeds to steps 2255 and 2270.

In step 2255, the method 2200 includes generating user guidance 2260 that the standard plane(s) have been detected and to end the follow-up scan(s). This user guidance 2260 can then be shown on the display 132.

In step 2270, the method 1600 includes performing an automatic measurement, or receiving a user input performing a manual measurement, of one or more anatomies used for GA determination in the detected standard planes. Automatic measurement is described for example in International Publication No. WO2024104857A1, filed November 8, 2023, entitled "Automatic measurement point detection for anatomy measurement in anatomical images", incorporated by reference as though fully set forth herein. Execution then proceeds to step 2275.

In step 2275, the method 2200 includes determining the gestational age 2280 using the automatic or manual measurement of the one or more anatomies. The gestational age 2280 can then be shown on the display 132. The method 2200 is now complete.

As will be readily appreciated by those having ordinary skill in the art after becoming familiar with the teachings herein, the ultrasound sweep standard plane detection system advantageously permits untrained and minimally trained users to perform an ultrasound blind sweep protocol to gather anatomical images of high quality that can be used to determine the gestational age of a fetus. This may result in higher accuracy and higher clinician trust in the results, while potentially improving health outcomes and/or decreasing the total cost of care.

The systems, methods, and devices described herein may be applicable in point of care and handheld ultrasound use cases such as with the Philips Lumify system. The ultrasound sweep standard plane detection system can be used for any handheld imaging applications, including but not limited to obstetrics, lung imaging, and echocardiography. The ultrasound sweep standard plane detection system could be deployed on handheld mobile ultrasound devices, and on portable or cart-based ultrasound systems. The ultrasound sweep standard plane detection system can be used in a variety of settings including emergency departments, ambulances, accident sites, and homes. The applications could also be expanded to other settings.

The invention is detectable from its functionality and output such as user instructions to go to a particular part of the mother's abdomen and do a series of acquisitions until a standard plane is found, or a prompt on screen to stop acquiring once standard plane is found, with gestational age estimation then being output on the screen.

A number of variations are possible on the examples and aspects described above. For example, the systems, methods, and devices described herein are not limited to obstetric ultrasound applications. Rather, the same technology can be applied to images of other organs or anatomical systems such as the lungs, heart, brain, digestive system, vascular system, etc. Furthermore, the technology disclosed herein is also applicable to other medical imaging modalities where 3D data are available, such as other ultrasound applications, camera-based videos, X-ray videos, and 3D volume images, such as computer aided tomography (CT) scans, magnetic resonance imaging (MRI) scans, optical coherence tomography (OCT) scans, or intravascular ultrasound (IVUS) pullback sequences. The technology described herein can be used in a variety of settings.

Accordingly, the logical operations making up the aspects of the technology described herein are referred to variously as operations, steps, objects, layers, elements, components, algorithms, or modules. Furthermore, it should be understood that these may occur or be performed or arranged in any order, unless explicitly claimed otherwise or a specific order is inherently necessitated by the claim language.

All directional references e.g., upper, lower, inner, outer, upward, downward, left, right, lateral, front, back, top, bottom, above, below, vertical, horizontal, clockwise, counterclockwise, proximal, and distal are only used for identification purposes to aid the reader's understanding of the claimed subject matter, and do not create limitations, particularly as to the position, orientation, or use of the ultrasound sweep standard plane detection system. Connection references, e.g., attached, coupled, connected, joined, or "in communication with" are to be construed broadly and may include intermediate members between a collection of elements and relative movement between elements unless otherwise indicated. As such, connection references do not necessarily imply that two elements are directly connected and in fixed relation to each other. The term "or" shall be interpreted to mean "and/or" rather than "exclusive or." The word "comprising" does not exclude other elements or steps, and the indefinite article "a" or "an" does not exclude a plurality. Unless otherwise noted in the claims, stated values shall be interpreted as illustrative only and shall not be taken to be limiting.

The above specification, examples and data provide a complete description of the structure and use of exemplary aspects of the ultrasound sweep standard plane detection system as defined in the claims. Although various aspects of the claimed subject matter have been described above with a certain degree of particularity, or with reference to one or more individual aspects, those skilled in the art could make numerous alterations to the disclosed aspects without departing from the spirit or scope of the claimed subject matter.

Still other aspects are contemplated. It is intended that all matter contained in the above description and shown in the accompanying drawings shall be interpreted as illustrative only of particular aspects and not limiting. Changes in detail or structure may be made without departing from the basic elements of the subject matter as defined in the following claims.

## Claims

1. An apparatus, comprising:
a processor circuit configured for communication with an ultrasound probe, wherein the processor circuit is configured to:
control the ultrasound probe to obtain a first plurality of ultrasound image frames during one or more sweeps of a blind sweep protocol on a patient with a pregnancy;
determine, based on the first plurality of ultrasound image frames, a location of the patient to perform a follow-up scan with the ultrasound probe, wherein the location is associated with a first fetal anatomy used to determine a gestational age of a fetus;
control the ultrasound probe to obtain a second plurality of ultrasound image frames during the follow-up scan at the location;
identify an ultrasound image frame in the second plurality comprising a pre-defined imaging plane of the first fetal anatomy;
determine the gestational age using the ultrasound image frame; and
output, to a display in communication with the processor, the gestational age.

2. The apparatus of claim 1,
wherein, to determine the location of the patient to perform the follow-up scan, the processor circuit is configured to:
provide the plurality of ultrasound image frames as an input to a deep learning network trained to detect a plurality of fetal anatomies; and
, as an output of the deep learning network, a plurality of detections of the plurality of fetal anatomies within the plurality of ultrasound image frames,
wherein the first fetal anatomy comprises one of the plurality of fetal anatomies,
wherein the plurality of detections comprises a detection of the first fetal anatomy, and
wherein the location comprises a location of the detection of the first fetal anatomy.

3. The apparatus of claim 2, wherein the processor circuit is configured to:
output, to a display in communication with the processor circuit, a user instruction to perform the follow-up scan with the ultrasound probe at the location.

4. The apparatus of claim 3,
wherein processor circuit is configured to generate a map of the plurality of detections, and
wherein the user instruction to the user comprises the map;
and optionally wherein the map comprises a plurality of regions that are visually distinguished from one another and representative of different fetal anatomies.

5. The apparatus of any of claims 1-4,0
wherein the processor is configured to output a user instruction to perform a plurality of movements with the ultrasound probe during the follow-up scan,
wherein the plurality of movements comprises at least one of rocking the ultrasound, fanning the ultrasound probe, or rotating the ultrasound probe.

6. The apparatus of claim 5, wherein the one or more sweeps of the blind sweep protocol do not include the at least one of rocking the ultrasound probe, fanning the ultrasound probe, or rotating the ultrasound probe.

7. The apparatus of any of claims 1-6, wherein, to identify the ultrasound image frame comprising the pre-defined imaging plane, the processor circuit is configured to:
provide the second plurality of ultrasound image frames as an input to a deep learning network trained to detect one or more pre-defined imaging planes; and
generate, as an output of the deep learning network, a detection that the ultrasound image comprises the pre-defined imaging plane.

8. The apparatus of any of claims 1-7,
wherein, in response to identification of the ultrasound image frame, the processor circuit is configured to output a user instruction comprising at least one of an indication that the pre-defined imaging plane has been found or guidance to end the follow-up scan.

9. The apparatus of any of claims 1-8, wherein the first fetal anatomy comprises one of a fetal head, a fetal abdomen, or a fetal femur.

10. The apparatus of claim 9, wherein, to determine the gestational age, the processor is configured to use:
a measurement of a head circumference or a measurement of a biparietal diameter in the ultrasound image frame, when the first fetal anatomy comprises the fetal head;
a measurement of an abdominal circumference in the ultrasound image frame, when the first fetal anatomy comprises the fetal abdomen; or
a measurement of a femur length in the ultrasound image frame, when the first fetal anatomy comprises the fetal femur.

11. The apparatus of claim 10, wherein the processor circuit is configured to automatically perform at least one the measurement of the head circumference, the measurement of the biparietal diameter, the measurement of the abdominal circumference, or the measurement of the femur length.

12. The apparatus of any of claims 1-11, wherein the processor circuit is configured to:
determine, based on the first plurality of ultrasound image frames, a further location of the patient to perform a further follow-up scan with the ultrasound probe, wherein the further location is associated with a different, second fetal anatomy used to determine the gestational age;
control the ultrasound probe to obtain a third plurality of ultrasound image frames during the further follow-up scan at further location;
identify a further ultrasound image frame in the third plurality comprising a pre-defined imaging plane of the second fetal anatomy; and
determine the gestational age using the further ultrasound image frame,
and optionally wherein the first fetal anatomy and the second fetal anatomy each comprises a different one of: a fetal head, a fetal abdomen, or a fetal femur.

13. The apparatus of any of claims 1-12, further comprising the ultrasound probe.

14. A method for determining a gestational age of a fetus using ultrasound data from blind abdominal imaging sweeps, comprising:
controlling an ultrasound probe to obtain a first plurality of ultrasound image frames during one or more sweeps of a blind sweep protocol on a patient with a pregnancy;
determining, based on the first plurality of ultrasound image frames, a location of the patient to perform a follow-up scan with the ultrasound probe, wherein the location is associated with a first fetal anatomy used to determine a gestational age of a fetus;
controlling the ultrasound probe to obtain a second plurality of ultrasound image frames during the follow-up scan at the location;
identifying an ultrasound image frame in the second plurality comprising a pre-defined imaging plane of the first fetal anatomy;
determining the gestational age using the ultrasound image frame; and
outputting, to a display, the gestational age.

15. A non-transitory computer-readable medium storing instructions that, when executed by a processor, cause the processor to perform the method of claim 14.
